# EUROPEAN PATENT APPLICATION

(11) **EP 3 345 549 A1**
(43) Date of publication of application: **11.07.2018**
(21) Application number: 17205635.0
(22) Date of filing: 06.12.2017
(51) Int. Cl.: A61B 8/00, G01S 15/89, G01S 7/52

(54) **PROBE FOR ULTRASONIC DIAGNOSTIC APPARATUS**

(30) Priority: 14.12.2016 KR 20160170415
(71) Applicant: Samsung Medison Co., Ltd., Hongcheon-gun, Gangwon-do, 25108 (KR)
(72) Inventor: CHOI, Kyung-moo, 17103 Gyeonggi-do (KR); KIM, Young-il, 16417 Gyeonggi-do (KR)
(74) Representative: D'Halleweyn, Nele Veerle Trees Gertrudis

(57) **Abstract**

Provided is a probe for an ultrasonic diagnostic apparatus, including a transducer module for transmitting or receiving ultrasound waves. The probe includes: a plurality of ultrasonic elements arranged in a two-dimensional (2D) array; a plurality of first electrical circuit boards located below the plurality of ultrasonic elements, spaced apart from one another by a predetermined distance along columns in one direction of the 2D array, and electrically connected to the plurality of ultrasonic elements; and a support frame including a plurality of slots into which the plurality of electrical circuit boards are inserted for support.

## Description

### CROSS-REFERENCE TO RELATED APPLICATION

This application claims the benefit of Korean Patent Application No. 10-2016-0170415, filed on December 14, 2016, in the Korean Intellectual Property Office, the disclosure of which is incorporated herein in its entirety by reference.

### BACKGROUND

### 1. Field

The present disclosure relates to probes for ultrasonic diagnostic apparatuses capable of obtaining an ultrasound image for use in diagnosis of a disease of an object.

### 2. Description of the Related Art

An ultrasonic diagnostic apparatus transmits ultrasound waves into an object, i.e., a living body such as a person or an animal, displays a cross-sectional image of tissue in the object by detecting echo signals reflected from the object, and provides information necessary for diagnosing a disease of the object. The ultrasonic diagnostic apparatus includes a probe for transmitting ultrasound waves into and receiving echo signals from the object. The probe includes ultrasonic transducers mounted therein for converting electrical signals into ultrasound signals or vice versa.

A plurality of ultrasonic elements, each including an ultrasonic transducer, are arranged in a two-dimensional (2D) array. A plurality of electrical circuit boards are respectively positioned below and electrically connected to the ultrasonic elements in a 2D array. When the ultrasonic elements and the electrical circuit boards mounted within a narrow housing are connected to one another, deformation of an electrical circuit board may occur during bonding, or misalignment between an ultrasonic element and a corresponding electrical circuit board may occur, thereby causing noise or interference among signals.

### SUMMARY

Provided are probes for an ultrasonic diagnostic apparatus capable of obtaining an ultrasound image for use in diagnosis of a disease of an object.

Additional aspects will be set forth in part in the description which follows and, in part, will be apparent from the description, or may be learned by practice of the presented embodiments.

According to an aspect of an embodiment, a probe for an ultrasonic diagnostic apparatus includes: a plurality of ultrasonic elements arranged in a two-dimensional (2D) array; a plurality of first electrical circuit boards located below the plurality of ultrasonic elements, spaced apart from one another by a predetermined distance along columns in one direction of the 2D array, and electrically connected to the plurality of ultrasonic elements; and a support frame including a plurality of slots into which the plurality of electrical circuit boards are inserted for support.

Each of the plurality of first electrical circuit boards may include a plurality of electrode lines spaced apart from one another by a predetermined distance along a direction that is different from the one direction of the 2D array

The plurality of first electrical circuit boards may be rigid printed circuit boards (PCBs).

Each of the plurality of first electrical circuit boards may further include a ground layer electrically insulated from the plurality of electrode lines.

An extendable pad may be provided at at least one of two opposite ends of each of the plurality of electrode lines and have a cross-sectional area greater than that of the electrode line.

Each of the plurality of slots may have a width less than or equal to 200 µm.

The support frame may further include border portions located between the plurality of slots, each border portion having a width less than or equal to 100 µm.

The support frame may include at least one of silicon, glass, and crystal materials.

The support frame may be formed using a micro electro-mechanical system (MEMS) technology.

The support frame may be formed as a plurality of support frames spaced apart from one another along a longitudinal direction of the plurality of first electrical circuit boards.

The probe may further include contact layers positioned between the plurality of first electrical circuit boards.

Each of the contact layers may include an epoxy adhesive material.

Each of the contact layers may include epoxy resin or hafnium oxide.

The probe may further include an acoustic backing member positioned between the plurality of ultrasonic elements and the plurality of first electrical circuit boards and preventing propagation of ultrasound waves to the plurality of first electrical circuit boards.

The probe may further include at least one conductive connecting portion penetrating the acoustic backing member and respectively corresponding to the plurality of ultrasonic elements and the plurality of electrode lines.

Each of the plurality of ultrasonic elements may include a transducer for transmitting or receiving an ultrasonic wave and an acoustic reflective layer positioned on a rear surface of the transducer and reflecting an ultrasonic wave transmitted to the rear of the transducer.

### BRIEF DESCRIPTION OF THE DRAWINGS

These and/or other aspects will become apparent and more readily appreciated from the following description of the embodiments, taken in conjunction with the accompanying drawings, in which reference numerals denote structural elements:
FIG. 1A is a block diagram of a configuration of an ultrasonic diagnostic apparatus according to an embodiment;
FIG. 1B illustrates an ultrasonic diagnostic apparatus according to an embodiment;
FIG. 2A is an internal view of an ultrasonic probe according to an embodiment;
FIG. 2B illustrates arrangement of ultrasonic elements according to an embodiment;
FIG. 3 is a schematic diagram of ultrasonic elements and an acoustic backing member, according to an embodiment;
FIG. 4A is an exploded perspective view of a first electrical circuit board according to an embodiment;
FIG. 4B is a front view of the first electrical circuit board of FIG. 4A;
FIG. 5A is a perspective view of first electrical circuit boards and a support frame, according to an embodiment;
FIG. 5B is a side view of the first electrical circuit boards and the support frame of FIG. 5A;
FIG. 6 is a plan view of a support frame according to an embodiment;
FIG. 7 is a side view of first electrical circuit boards and a support frame, according to another embodiment; and
FIG. 8 is a side view of first electrical circuit boards and a support frame, according to another embodiment.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

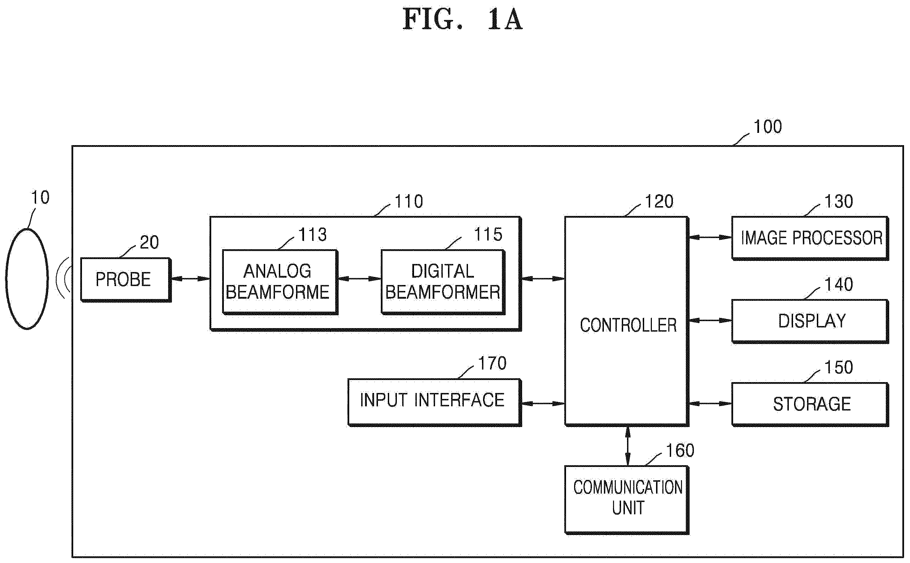

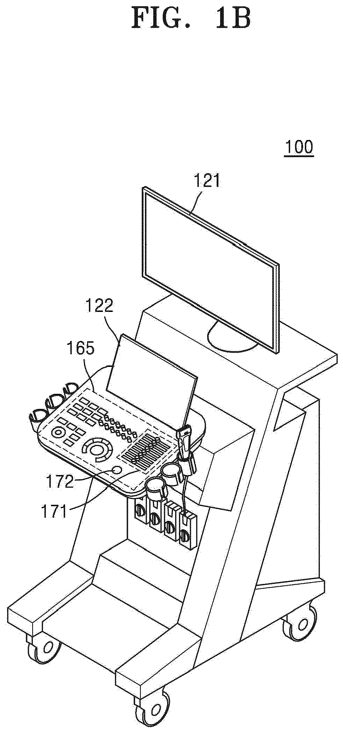

FIG. 1A is a block diagram illustrating a configuration of an ultrasound diagnosis apparatus 100, i.e., a diagnostic apparatus, according to an exemplary embodiment. FIGS. 1B is diagram illustrating ultrasound diagnosis apparatus according to an exemplary embodiment.

Referring to FIG. 1A, the ultrasound diagnosis apparatus 100 may include a probe 20, an ultrasound transceiver 110, a controller 120, an image processor 130, one or more displays 140, a storage 150, e.g., a memory, a communication unit 160, i.e., a communication device or an interface, and an input interface 170.

In the present embodiment, the probe 20 may include a plurality of transducers. The transducers are arranged in two dimensions (2D), forming a 2D transducer array.

For example, the 2D transducer array may include a plurality of sub-arrays arranged in a first direction, each of the sub-arrays including a plurality of transducers arranged in a second direction that is different from the first direction.

The ultrasound transceiver 110 may include an analog beamformer 113 and a digital beamformer 115. Although FIG. 1A illustrates that the ultrasound transceiver 110 and the probe 20 are provided as being separate from each other, the probe 20 according to the present exemplary embodiment may include the entire ultrasound transceiver 110 or a part of the ultrasound transceiver 110. For example, the probe 20 may include one or both of the analog beamformer 113 and the digital beamformer 115.

The controller 120 may calculate a time delay value for digital beamforming with respect to the sub-arrays included in the 2D transducer array. Also, the controller 120 may calculate a time delay value for analog beamforming for each of the transducers included in any one sub-array of the sub-arrays.

The controller 120 may control the analog beamformer 113 and the digital beamformer 115 to form a transmission signal to be applied to each of the transducers, according to the time delay values for analog beamforming and digital beamforming.

Also, the controller 120 may control the analog beamformer 113 to add signals received from the transducers for each sub-array, according to the time delay value for analog beamforming. Also, the controller 120 may control the ultrasound transceiver 110 to perform analog to digital conversion of the signals added for each sub-array. Also, the controller 120 may control the digital beamformer 115 to generate ultrasound data by adding the digitized signals according to the time delay value for digital beamforming.

The image processor 130 generates an ultrasound image by using generated ultrasound data.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communication unit 160 and may be connected to external apparatuses, for example, servers, medical apparatuses, and portable devices such as smart phones, tablet personal computers (PCs), wearable devices, etc., via the communication unit 160.

The communication unit 160 may include at least one element capable of communicating with the external apparatuses. For example, the communication unit 160 may include at least one among a short-range communication module, a wired communication module, and a wireless communication module.

The communication unit 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communication unit 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communication unit 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

An example of the ultrasound diagnosis apparatus 100 according to the present exemplary embodiment is described below with reference to FIGS. 1B.

Referring to FIGS. 1B, the ultrasound diagnosis apparatus 100 may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUI), thereby receiving user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

The ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Hereinafter, a probe for an ultrasonic diagnostic apparatus will be described in more detail with reference to the accompanying drawings.

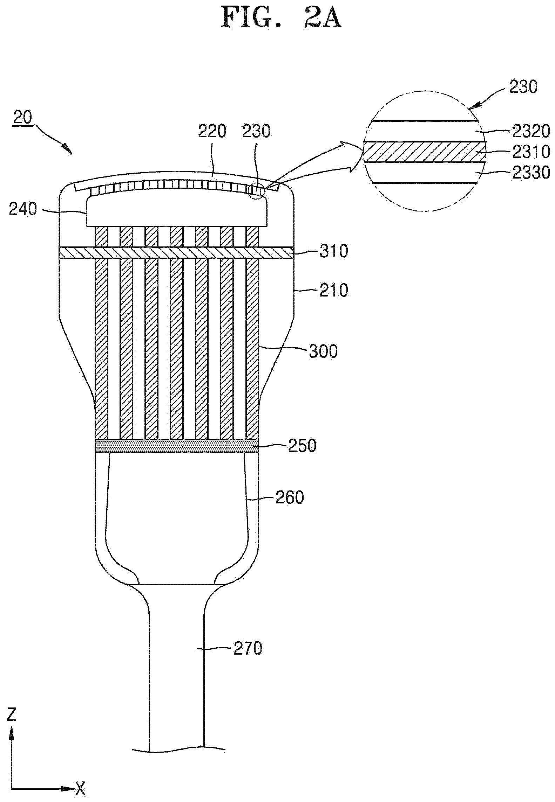

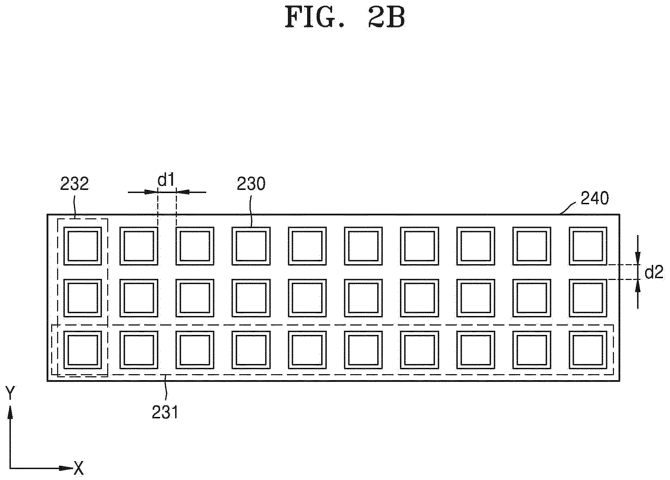

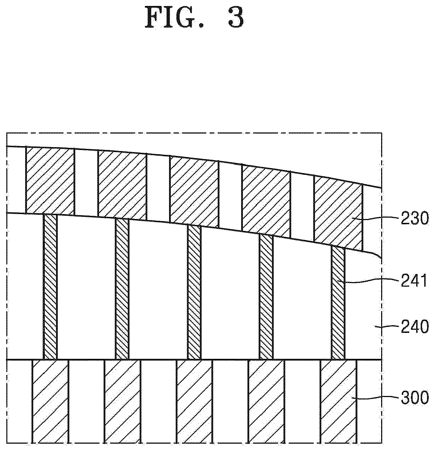

FIG. 2A is an internal view of a probe 20 for an ultrasonic diagnostic apparatus according to an embodiment, and FIG. 2B illustrates arrangement of ultrasonic elements 230 according to an embodiment. FIG. 3 is a schematic diagram of ultrasonic elements 230 and an acoustic backing member 240 according to an embodiment.

Referring to FIGS. 2A and 2B, the probe 20 may include an acoustic lens 220 positioned at one distal end of a probe housing 210, the ultrasonic elements 230 located adjacent to the acoustic lens 220, the acoustic backing member 240 having one surface on which the ultrasonic elements 230 are seated and the other surface to which first electrical circuit boards 300 are mounted, the first electrical circuit boards 300, each having one surface attached to the other surface of the acoustic backing member 240, which serve as electrical connecting elements, a support frame 310 for supporting the first electrical circuit boards 300, a second electronic circuit 250 electrically connected to the first electrical circuit boards 300 and attached to the other surface of each of the first electrical circuit boards 300, and a connecting line 260 for transmitting a signal output from the second electronic circuit 250 to the controller (120 of FIG. 1A).

The ultrasonic elements 230, the acoustic backing member 240, the first electrical circuit boards 300, the support frame 310, the second electronic circuit 250, and the connecting line 260 may be mounted inside the probe housing 210. A cable 270 may be fixedly attached to or detached from the other distal end of the probe housing 210.

The probe housing 210 may protect various components of the probe 20 against external shock while stably keeping them in position. The probe housing 210 may be formed of various metals or synthetic resin and have various shapes depending on the purpose of using the probe 20 or the type of the object (10 of FIG. 1A) or a target portion.

The acoustic lens 220 may focus or diverge ultrasound waves or other sound waves. The acoustic lens 220 may focus ultrasound waves generated by the ultrasonic elements 230 on the object 10, and may be formed of glass or synthetic fiber.

The ultrasonic elements 230 may be seated on the one surface of the acoustic backing member 240 to be in contact with or adjacent to the acoustic lens 220. According to an embodiment, the probe 10 may include a plurality of ultrasonic elements 230. When the probe 20 includes the plurality of ultrasonic elements 230, the ultrasonic elements 230 may be arranged two-dimensionally to form a 2D ultrasonic element array as shown in FIG. 2B. For example, the 2D ultrasonic element array may include a first sub-array 231 containing a plurality of ultrasonic elements 230 arranged in a first (X) direction and a second sub-array 232 containing a plurality of ultrasonic elements 230 arranged in a second (Y) direction that is different from the first (X) direction. In this case, the plurality of ultrasonic elements 230 may be respectively spaced apart from one another by a first distance d1 along the first (X) direction and by a second distance d2 along the second (Y) direction.

According to an embodiment, the plurality of ultrasonic elements 230 may respectively include a plurality of transducers 2310. For example, the transducers 2310 may be magnetostrictive ultrasonic transducers that utilize a magnetostrictive effect exhibited by a magnetic material, or piezoelectric ultrasonic transducers that utilize a piezoelectric effect exhibited by a piezoelectric material. Furthermore, the transducers 2310 may be capacitive micromachined ultrasonic transducers (hereinafter abbreviated as "cMUTs") that utilize vibration of several hundreds or thousands of micromachined thin films to transmit or receive ultrasound waves. In addition, the transducers 2310 may be piezoelectric micromachined ultrasonic transducers (pMUTs), each consisting of several hundreds or thousands of piezoelectric thin films using lead zirconate titanate (Pb(Zr, Ti)O₃) (PZT) or single-crystal lead magnesium niobate (Pb(Mg_{1/3}Nb_{2/3})O₃)-lead titanate (PbTiO₃) (PMN-PT) unlike cMUTs. It is assumed hereinafter that the transducers 2310 are piezoelectric ultrasonic transducers. However, the transducers 2310 used in the probe 20 are not limited to piezoelectric ultrasonic transducers.

According to an embodiment, when the ultrasonic elements 230 are arranged in a 2D array, and the transducers 2310 respectively included in the ultrasonic elements 230 are formed as piezoelectric ultrasonic transducers, one piezoelectric material may be divided into a plurality of regions to form a 2D array of the transducers 2310. For example, the transducers 2310 may be fabricated by dicing a piezoelectric material elongated in a width direction. However, a method of fabricating the transducers 2310 by dividing a piezoelectric material is not limited thereto, and the transducers 2310 may be manufactured using other various methods, e.g., by pressing a piezoelectric material using a metal mold. The piezoelectric material may be a material exhibiting a piezoelectric effect, such as a piezoelectric ceramic material, a single crystal, or a composite piezoelectric material made by combining the piezoelectric ceramic material with polymer. However, embodiments are not limited thereto.

A matching layer 2320 may be provided on a front surface of each transducer 2310 and match acoustic impedances between the transducer 2310 and the object 10 so that an ultrasound signal generated by the transducer 2310 may be efficiently transmitted to the object 10. For this purpose, the matching layer 2310 may include a material having an intermediate value of acoustic impedances of the transducer 2310 and the object 10, such as a glass or resin material, but embodiments are not limited thereto.

An acoustic reflective layer 2330 may be positioned on a rear surface of the transducer 2310 to amplify vibrational energy generated by the transducer 2310. For example, the acoustic reflective layer 2330 may amplify ultrasound waves transmitted from the transducer 2310 by reflecting vibrational energy transmitted in a backward direction back again in a forward direction. In other words, the acoustic reflective layer 2330 may increase acoustic pressure amplitude of ultrasound waves transmitted from the transducer 2310. Furthermore, the acoustic reflective layer 2330 may include a material having an acoustic impedance higher than that of the transducer 2310.

The acoustic backing member 240 may be positioned at a rear surface of the acoustic reflective layer 2330 to reduce a pulse width of ultrasound waves by suppressing free vibration of the transducer 2310 and to prevent image distortion by blocking unnecessary propagation of ultrasound waves to the rear of the transducer 2310. For example, depending on the design, acoustic impedances of the acoustic backing member 240 may be combined in various ways, e.g., by making one acoustic impedance equal to or higher than another. Thus, it is possible to easily obtain combinations of acoustic impedances that permit absorption or reflection of ultrasound waves.

Referring to FIG. 3, at least one conductive connecting portion 241 may be formed in the acoustic backing member 240 to penetrate the acoustic backing member 240 from one surface to the other surface. In this case, "the other surface" refers to a surface opposite to the one surface of the acoustic backing member 240. The at least one conductive connecting portion 241 may pass through the acoustic backing member 240 to be exposed to outside on both the one and the other surfaces thereof.

The at least one conductive connecting portion 241 may be formed of an electrically conductive material that allows the flow of an electric current. Examples of the electrically conductive material may include various metals such as copper (Cu) and gold (Au). Thus, the at least one conductive connecting portion 241 may transmit electrical signals output from the ultrasonic elements 230 to the first electrical circuit boards 300 or transmit electrical signals output from the first electrical circuit boards 300 to the ultrasonic elements 230.

The ultrasonic elements 230 may be seated on the one surface of the acoustic backing member 240. The one surface of the acoustic backing member 240 may be formed as a planar surface. Alternatively, according to an embodiment, the one surface of the acoustic backing member 240 may be formed as a curved surface having a predetermined curvature. Furthermore, the first electrical circuit boards 300 may be mounted to the other surface of the acoustic backing member 240. Similarly, the other surface of the acoustic backing member 240 may be formed as a planar surface or curved surface having a predetermined curvature.

Each of the first electrical circuit boards 300 is an electrical circuit module that is mounted to the other surface of the acoustic backing member 240 for electrical connection with its corresponding ultrasonic element 230. According to an embodiment, each of the first electrical circuit boards 300 may include a substrate, various circuits mounted on the substrate, and semiconductor chips or various components connected to the various circuits. According to an embodiment, the first electrical circuit board 300 may not include at least one of the substrate, various circuits, and semiconductor chips or various components.

As shown in FIG. 2A, the first electrical circuit boards 300 may be arranged behind a 2D array of the ultrasonic elements 230 and may be spaced apart from one another by a specific distance. Thus, according to an embodiment, the first electrical circuit boards 300 may be rigid printed circuit boards (PCBs) that are not easy to bend for alignment with the ultrasonic elements 230. In this case, "not easy to bend" does not mean that the rigid PCBs do not bend at all, but that they do not generally bend during normal use. However, embodiments are not limited thereto, and the first electrical circuit boards 300 may be rigid flexible PCBs or flexible PCBs as long as they can remain constantly aligned with the ultrasonic elements 230. Hereinafter, the first electrical circuit boards 300 and a support structure for the first electrical circuit boards 300 will be described in more detail, assuming that rigid PCBs are used as the first electrical circuit boards 300.

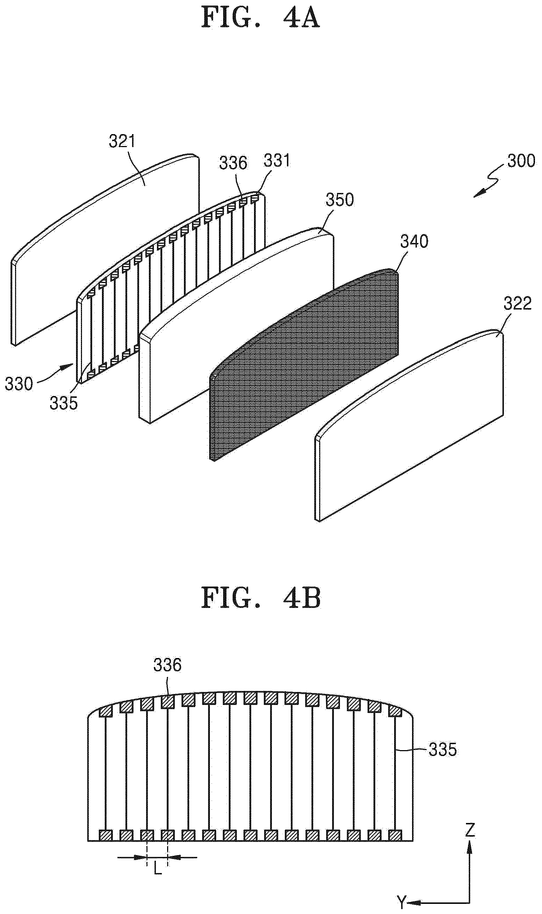

FIG. 4A is an exploded perspective view of a first electrical circuit board 300 according to an embodiment, and FIG. 4B is a front view of the first electrical circuit board 300 of FIG. 4A.

Referring to FIGS. 4A and 4B, according to an embodiment, the first electrical circuit board 300 may be formed as a multi-layered structure including first and second cover layers 321 and 322, a signal line layer 330, a ground layer 340, and a passivation layer 350 sandwiched between the signal line layer 330 and the ground layer 340. For example, the first and second cover layers 321 and 322 may each include an unbendable, rigid insulating material, and the passivation layer 350 may be a protective layer for preventing contact between the signal line layer 330 and the ground layer 340.

The signal line layer 330 may include electrode lines 335 located on an insulating substrate 331. According to an embodiment, the electrode lines 335 in the signal line layer 330 may be signal electrodes electrically connected to the ultrasonic elements 230 via the at least one conductive connecting portion 241.

According to an embodiment, each of the electrode lines 335 may extend in a third (Z) direction. In this case, the third (Z) direction is defined as a direction orthogonal to first (X) and second (Y) directions in which the ultrasonic elements (230 of FIG. 2B) are arranged two-dimensionally. In this case, one end of the electrode line 335 extending in the third (Z) direction is exposed through a front surface of the first electrical circuit board 300 to be electrically connected to the conductive connecting portion 241, and the other end thereof is exposed through a rear surface thereof to be electrically connected to the second electronic circuit 250. In this case, an extendable pad 336 may be provided at at least one of the two opposite ends of the electrode line 335 to improve contact between either of the two opposite ends and the conductive connecting portion 241 or the second electronic circuit 250. For example, the extendable pad 336 may have a cross-sectional area greater than that of the electrode line 335 and include a conductive material. Furthermore, the extendable pad 336 may be formed integrally with the electrode line 335 or be formed separately from the electrode line 335 to adhere thereto.

Furthermore, as shown in FIG. 4B, the signal line layer 330 may include a plurality of electrode lines 335, and the electrode lines 335 may be spaced apart from one another along a "direction of arrangement."

The "direction of arrangement" is defined as a direction in which the plurality of ultrasonic elements 230 are arranged in the form of an array. In other words, the electrode lines 335 may be spaced apart from one another along the second (Y) direction in which the ultrasonic elements 230 are arranged in a 2D array. Furthermore, in this case, the electrode lines 335 may be arranged in such a manner as to be spaced apart from one another by a distance L that is substantially equal to the second distance d2 by which the ultrasonic elements 230 are spaced apart from one another along the second (Y) direction. Thus, the electrode lines 335 may correspond one-to-one to the ultrasonic elements 230 along the second (Y) direction. However, the direction of arrangement of the electrode lines 335 is not limited to the second (Y) direction, and the electrode lines 335 may be spaced apart from one another along the first (X) direction in which the plurality of ultrasonic elements 230 are arranged in a 2D array.

The ground layer 340 may be a ground part opposing the signal line layer 330 with the passivation layer 350 interposed therebetween. As described above, when the first electrical circuit boards 300 are spaced apart from one another by a very small distance along a direction in which the ultrasonic elements 230 are arranged, crosstalk may occur among the electrode lines 335 located adjacent to one another along the direction of arrangement of the ultrasonic elements 230. In this case, by placing the ground layer 340 between the adjacent electrode lines 335, it is possible to prevent crosstalk, which may cause adverse effects among the electrode lines 335, and accordingly obtain a clearer image.

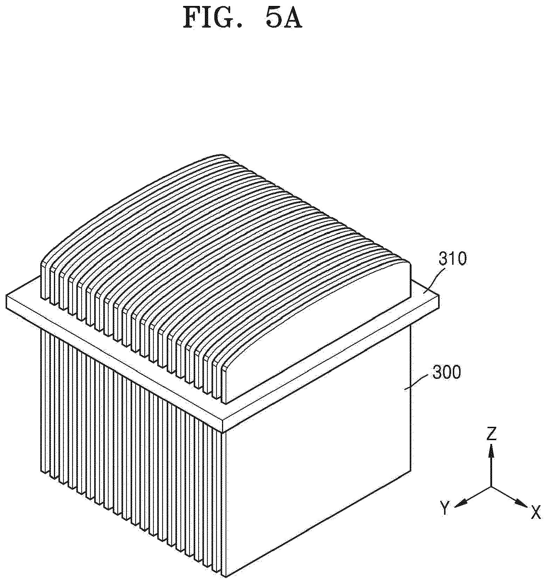

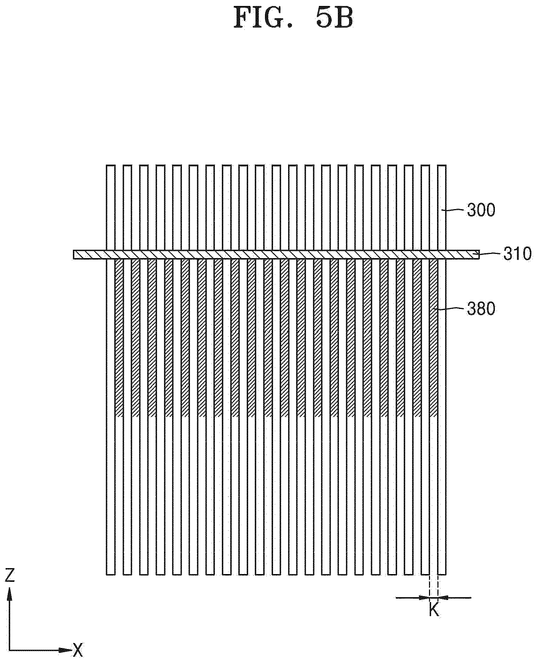

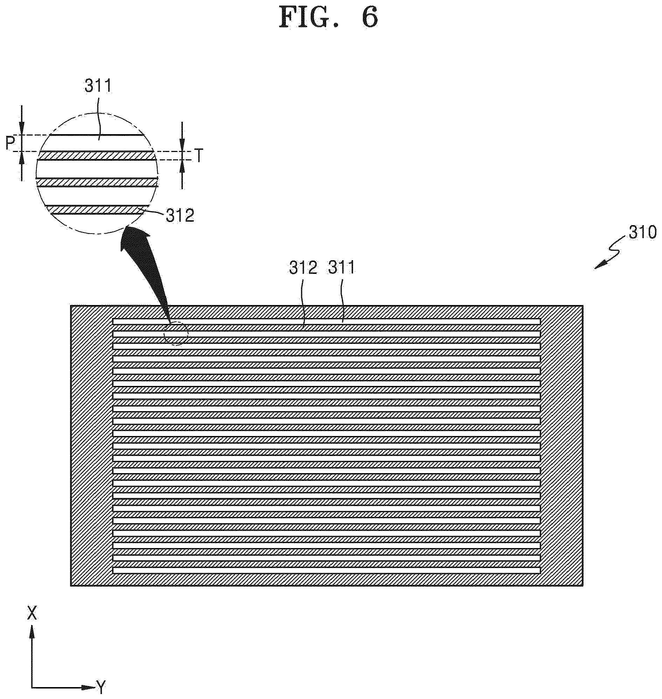

FIG. 5A is a perspective view of a plurality of first electrical circuit boards 300 and a support frame 310 according to an embodiment, and FIG. 5B is a side view of the first electrical circuit boards 300 and the support frame 310 of FIG. 5A. FIG. 6 is a plan view of a support frame 310 according to an embodiment.

Referring to FIGS. 5A and 5B, according to an embodiment, the first electrical circuit boards 300 may be arranged in such a manner as to be spaced apart from one another by a predetermined distance along a first (X) direction. In this case, the number of the first electrical circuit boards 300 may be determined to be substantially equal to the number of the ultrasonic elements 230 arranged along the first (X) direction as shown in FIG. 2B. Furthermore, the distance K along the first (X) direction for the first electrical circuit boards 300 may be substantially equal to the first distance d1 along the first (X) direction for the ultrasonic elements 230 shown in FIG. 2B. For example, the distance K corresponding to the first distance d1 may be on the order of several micrometers (µm), e.g., may be less than or equal to 100 µm. Thus, the first electrical circuit boards 300 may substantially correspond one-to-one to the ultrasonic elements 230 along the first (X) direction.

Furthermore, according to an embodiment, a contact layer 380 may be positioned between adjacent circuit boards among the first electrical circuit boards 300 to maintain a distance therebetween. For example, if a single support frame 310 is located on the first electrical circuit boards 300, a support force may not be strong enough to maintain a distance between adjacent circuit boards among the first electrical circuit boards 300. In this case, by placing the contact layer 380 between adjacent circuit boards of the first electrical circuit boards 300, it is possible to generate a sufficient support force for maintaining a distance therebetween. According to an embodiment, the contact layer 380 may include an epoxy adhesive material. Furthermore, the contact layer 380 may include epoxy resin, hafnium oxide such as hafnium oxide metal powder, etc., and accordingly may function as an additional acoustic backing member.

The support frame 310 may support the first electrical circuit boards 300 to correspond one-to-one to the ultrasonic elements 230. Referring to FIG. 6, according to an embodiment, the support frame 310 may be formed as a plate-like support member including a plurality of slots 311. The slots 311 may extend along a second (Y) direction, and the number of slots 311 may be determined depending on the number of first electrical circuit boards 300 to be supported.

Furthermore, according to an embodiment, each of the slots 311 formed in the support frame 310 may have a predetermined width P along a first (X) direction. For example, the predetermined width P may be substantially equal to a thickness of each of the first electrical circuit boards 300 so that the first electrical circuit board 300 may be inserted into one of the slots 311. A thickness of each of the first electrical circuit boards 300 respectively corresponding to the ultrasonic elements 230 in a 2D array may be on the order of several µm, e.g., may be less than or equal to 200 µm. Accordingly, the predetermined width P of each of the slots 311 may also be on the order of several µm, e.g., may be less than or equal to 200 µm. However, embodiments are not limited thereto, and if a support portion for supporting the first electrical circuit boards 300 is provided in the support frame 310, the slots 311 may have any of various widths P that are large enough to allow insertion of the first electrical circuit board 300 thereinto.

Furthermore, according to an embodiment, border portions 312 may be located between the slots 311 in the support frame 310 to determine a boundary therebetween, each having a predetermined width T along the first (X) direction. For example, the predetermined width T of each of the border portions 312 may be substantially equal to the distance K by which the first electrical circuit boards 300 are spaced apart from one another. According to an embodiment, the distance K between adjacent ones of the first electrical circuit boards 300 may be on the order of several µm, e.g., may be less than or equal to 100 µm. Thus, the width T of each border portion 312 may also be on the order of several µm, e.g., may be less than or equal to 100 µm.

Furthermore, since the support frame 310 may include the slots 311 and the border portions 312 having widths of the order of several µm, the support frame 310 may be implemented using a micro electro-mechanical system (MEMS) technology that allows fabrication with a tolerance of a few µm. Thus, the support frame 310 may include materials suitable for MEMS, such as silicon, glass, and crystal. However, embodiments are not limited thereto, and the support frame 310 may be fabricated by another process or may include another material as long as the other process or material is used to form the support frame 310 including the slots 311 and the border portions 312 having widths of the order of several µm, which is substantially the same as that fabricated using the MEMS technology.

By implementing the support frame 310 with a MEMS technology, the support frame 310 including the slots 311 and the border portions 312 having widths of the order of several µm may mechanically support the first electrical circuit boards 300 spaced apart from one another by a minute distance K. Thus, even when the ultrasonic elements 230 undergo high temperature bonding, deformation of the first electrical circuit boards 300 may be prevented. Furthermore, when the first electrical circuit boards 300 are formed as rigid PCBs and are supported using the support frame 310, it is possible to constantly maintain alignment between the ultrasonic elements 230 and the first electrical circuit boards 300 and accordingly prevent interference among signals or occurrence of noise.

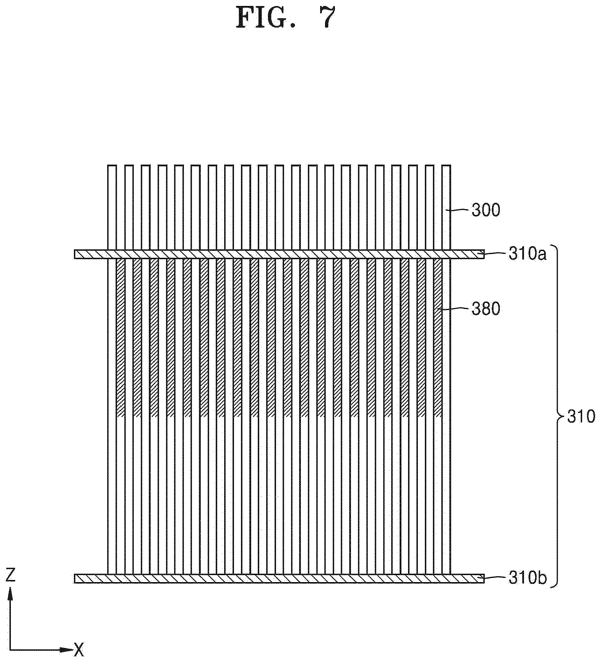

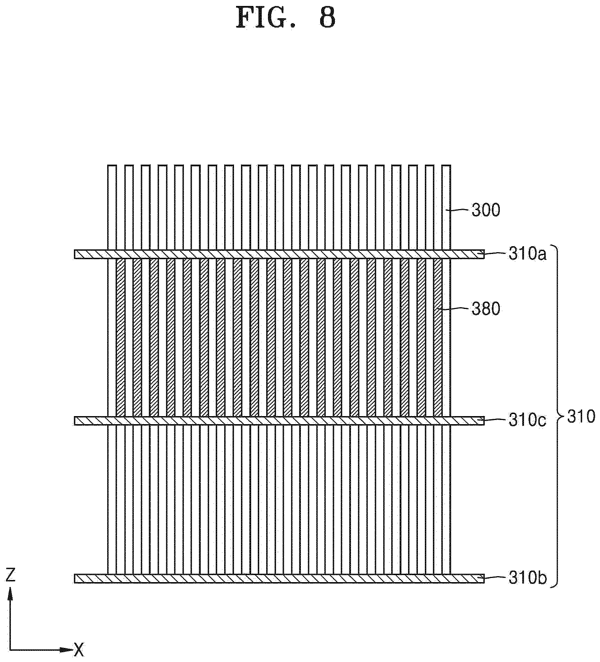

FIG. 7 is a side view of first electrical circuit boards 300 and support frames 310a and 310b according to another embodiment, and FIG. 8 is a side view of first electrical circuit boards 300 and support frames 310a through 310c according to another embodiment.

According to an embodiment, a support frame 310 may be implemented as a plurality of support frames. Referring to FIG. 7, the support frame 310 may be formed as first and second support frames 310a and 310b. For example, if the support frame 310 is formed as the first and second support frames 310a and 310b, the first and second support frames 310a and 310b may be located along a third (Z) direction, e.g., may be respectively located on upper and lower parts of the first electrical circuit boards 300 so that they are spaced apart from each other. In this case, unlike when the single support frame 310 is located on the first electrical circuit boards 300 as shown in FIG. 5B, a separate contact layer 380 may not be positioned between the first electrical circuit boards 300 shown in FIG. 7. However, embodiments are not limited thereto, and the contact layer 380 may be disposed to apply an additional support force to the first electrical circuit boards 300.

Furthermore, referring to FIG. 8, according to an embodiment, a support frame 310 may be implemented as first through third support frames 310a through 310c. For example, if the support frame 310 is formed as the first through third support frames 310a through 310c, the first through third support frames 310a through 310c may be located along the third (Z) direction, e.g., may be respectively located on upper, middle, and lower parts of the first electrical circuit boards 300 so that they are spaced apart from one another. In this case, the number of support frames may be determined depending on a support force for supporting the first electrical circuit boards 300 and an inner space of the probe housing (210 of FIG. 2A), and three or more support frames may be provided to support the first electrical circuit boards 300.

According to embodiments, in a probe for an ultrasonic diagnostic apparatus including ultrasonic elements arranged in a 2D array, a support frame may be provided for mechanically supporting a plurality of electrical circuit boards respectively connected to the ultrasonic elements. Due to the presence of the support frame, even when high temperature bonding is performed on the ultrasonic elements, deformation of the electrical circuit boards may be prevented.

Furthermore, by supporting the electrical circuit boards through the support frame, it is possible to more constantly maintain alignment between the ultrasonic elements in the 2D array and the electrical circuit boards and accordingly prevent interference among signals or occurrence of noise.

While one or more embodiments have been described with reference to the figures, it will be understood by those of ordinary skill in the art that various changes in form and details may be made therein without departing from the spirit and scope of the present disclosure as defined by the following claims. Accordingly, the above embodiments and all aspects thereof are examples only and are not limiting.

## Claims

1. A probe for an ultrasonic diagnostic apparatus, the probe comprising:
a plurality of ultrasonic elements arranged in a two-dimensional (2D) array;
a plurality of first electrical circuit boards located below the plurality of ultrasonic elements, spaced apart from one another by a predetermined distance along columns in one direction of the 2D array, and electrically connected to the plurality of ultrasonic elements; and
a support frame including a plurality of slots into which the plurality of electrical circuit boards are inserted for support.

2. The probe of claim 1, wherein each of the plurality of first electrical circuit boards comprises a plurality of electrode lines spaced apart from one another by a predetermined distance along a direction that is different from the one direction of the 2D array

3. The probe of claim 2, wherein the plurality of first electrical circuit boards are rigid printed circuit boards (PCBs).

4. The probe of claim 3, wherein each of the plurality of first electrical circuit boards further comprises a ground layer electrically insulated from the plurality of electrode lines.

5. The probe of claim 3, wherein an extendable pad is provided at at least one of two opposite ends of each of the plurality of electrode lines and has a cross-sectional area greater than that of the electrode line.

6. The probe of claim 1, wherein each of the plurality of slots has a width less than or equal to 200 µm.

7. The probe of claim 6, wherein the support frame further comprises border portions located between the plurality of slots, each border portion having a width less than or equal to 100 µm.

8. The probe of claim 7, wherein the support frame includes at least one of silicon, glass, and crystal materials.

9. The probe of claim 6, wherein the support frame is formed using a micro electro-mechanical system (MEMS) technology.

10. The probe of claim 6, wherein the support frame is formed as a plurality of support frames spaced apart from one another along a longitudinal direction of the plurality of first electrical circuit boards.

11. The probe of claim 1, further comprising contact layers positioned between the plurality of first electrical circuit boards.

12. The probe of claim 11, wherein each of the contact layers includes an epoxy adhesive material.

13. The probe of claim 11, wherein each of the contact layers includes epoxy resin or hafnium oxide.

14. The probe of claim 2, further comprising an acoustic backing member positioned between the plurality of ultrasonic elements and the plurality of first electrical circuit boards and preventing propagation of ultrasound waves to the plurality of first electrical circuit boards.

15. The probe of claim 14, further comprising at least one conductive connecting portion penetrating the acoustic backing member and respectively corresponding to the plurality of ultrasonic elements and the plurality of electrode lines.
